# EUROPEAN PATENT APPLICATION

(11) **EP 1 891 955 A1**
(43) Date of publication of application: **27.02.2008**
(21) Application number: 06291194.6
(22) Date of filing: 24.07.2006
(51) Int. Cl.: A61K 31/4745, A61P 19/02

(54) **Use of 1,2,3-substituted indolizine derivatives, inhibitors of FGFs, for the preparation of a medicament intended for the treatment of degenerative joint diseases**

(71) Applicant: Sanofi-Aventis, 75013 Paris (FR)
(72) Inventor: Bono, Francoise, c/o sanofi-aventis, 75013 Paris (FR); Rudolphi, Karl, c/o sanofi-aventis Deutschland, 65926 Frankfurt am Main (DE)
(74) Representative: Bouvet, Philippe

(57) **Abstract**

The present invention concerns the use of 1,2,3-substituted indolizine derivatives of formula (I), as defined in the description; 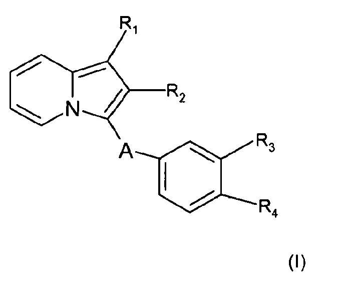 and in particular the 2-amino-5-[(1-methoxy-2-methylindolizin-3-yl)carbonyl]benzoic acid, and their pharmaceutically acceptable salts and solvates for the preparation of a medicament intended for the treatment of degenerative joint diseases, such as osteoarthritis, spondyloses, cartilage loss following joint trauma or a relatively long period of joint immobilization following meniscus or patella injuries or ligament ruptures, and chronic diseases of the locomotory apparatus such as inflammatory, immunological or metabolism-associated acute and chronic arthritides, arthropathies, myalgias and disturbances of bone metabolism.

## Description

The present invention relates to a novel use of 1,2,3-substituted indolizine derivatives, which are inhibitors of FGFs (basic fibroblast growth factors). More particularly, the invention relates to the use of FGFs receptor antagonists for the preparation of a medicament intended for the treatment of osteoarthritis.

1,2,3-substituted indolizine derivatives, which are potent antagonists of the binding of FGFs to its receptors, have been disclosed in international patent application WO 03/084956. It is disclosed that 1,2,3-substituted indolizine derivatives, and their salts, which are antagonists of FGFs receptors, may be useful in the treatment of carcinomas having a high degree of vascularization, such as carcinomas of the lung, breast, prostate and oesophagus, cancers which induce metastases, such as colon cancer and stomach cancer, melanomas, gliomas, lymphomas and leukaemias, in the treatment of cardiovascular diseases such as atherosclerosis, post-angioplasty restenosis, diseases linked to complications which appear following the fitting of endovascular prostheses and/or aorto-coronary artery by-pass surgery or other vascular transplants, cardiac hypertrophy, or vascular complications in diabetes such as diabetic retinopathies, in the treatment of chronic inflammatory diseases such as rheumatoid arthritis or IBDs, and in the treatment of achondroplasia (ACH), hypochondroplasia (HCH) and TD (thanatophoric dysplasia).
Furthermore, international patent application WO 2005/082457 discloses the use 1,2,3-substituted indolizine derivatives, which are antagonists of FGFs receptors, to treat diseases linked to pathological choroidal angiogenesis.

It has now been found surprisingly that 1,2,3-substituted indolizine derivatives, which are inhibitors of FGFs, in particular the 2-amino-5-[(1-methoxy-2-methylindolizin-3-yl)carbonyl]benzoic acid, and their pharmaceutically acceptable salts and solvates are useful in the treatment of degenerative joint diseases.

Hence, the present invention concerns the use of a 1,2,3-substituted indolizine derivative, and in particular the 2-amino-5-[(1-methoxy-2-methylindolizin-3-yl)carbonyl]benzoic acid, and its pharmaceutically acceptable salts and solvates, for the preparation of a medicament intended for the treatment of degenerative joint diseases, such as osteoarthritis, spondyloses, cartilage loss following joint trauma or a relatively long period of joint immobilization following meniscus or patella injuries or ligament ruptures, and chronic diseases of the locomotory apparatus such as inflammatory, immunological or metabolism-associated acute and chronic arthritides, arthropathies, myalgias and disturbances of bone metabolism.

The present invention concerns particularly the use of a 1,2,3-substituted indolizine derivative, and in particular the 2-amino-5-[(1-methoxy-2-methylindolizin-3-yl)carbonyl]benzoic acid, and its pharmaceutically acceptable salts and solvates, for the preparation of a medicament intended for the treatment of osteoarthritis.

The 1,2,3-substituted indolizine derivatives used in the invention correspond to the following formula (I): 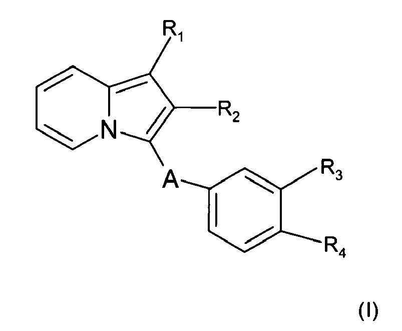 in which:
- R₁ represents a hydroxyl radical, a linear or branched alkoxy radical of 1 to 5 carbon atoms, a carboxyl radical, an alkoxycarbonyl radical of 2 to 6 carbon atoms or a radical of formula:
   - -NR₅R₆
   - -NH-SO₂-Alk
   - -NH-SO₂-Ph
   - -NH-CO-Ph
   - -N(Alk)-CO-Ph
   - -NH-CO-NH-Ph
   - -NH-CO-Alk
   - -NH-CO₂-Alk
   - -O-(CH₂)ₙ-cAlk
   - -O-Alk-COOR₇
   - -O-Alk-O-R₈
   - -O-Alk-OH
   - -O-Alk-C(NH₂):NOH
   - -O-Alk-NR₅R₆
   - -O-Alk-CN
   - -O-(CH₂)ₙ-Ph
   - -O-Alk-CO-NR₅R₆
   - -CO-NH-(CH₂)ₘ-COOR₇
   - -CO-NH-Alk
   in which
   - Alk represents an alkyl radical or a linear or branched alkylene radical of 1 to 5 carbon atoms,
   - cAlk represents a cycloalkyl radical of 3 to 6 carbon atoms,
   - n represents an integer from 0 to 5,
   - m represents an integer from 1 to 5,
   - R₅ and R₆, which are identical or different, each represent a hydrogen atom, a linear or branched alkyl radical of 1 to 5 carbon atoms or a benzyl radical,
   - R₇ represents a hydrogen atom or an alkyl radical of 1 to 5 carbon atoms,
   - R₈ represents an alkyl radical of 1 to 5 carbon atoms or a radical -CO-Alk,
   - Ph represents a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
- R₂ represents a hydrogen atom, an alkyl radical of 1 to 5 carbon atoms, an alkyl halide radical of 1 to 5 carbon atoms containing 3 to 5 halogen atoms, a cycloalkyl radical of 3 to 6 carbon atoms or a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
- A represents a radical -CO-, -SO- or -SO₂-,
- R₃ and R₄, which are identical or different, each represent a hydrogen atom, an alkoxy radical of 1 to 5 carbon atoms, an amino radical, a carboxyl radical, an alkoxycarbonyl radical of 2 to 6 carbon atoms, a hydroxyl radical, a nitro radical, a hydroxyamino radical, a radical of formula
   - -Alk-COOR₇
   - -NR₅R₆
   - -NH-Alk-COOR₇
   - -NH-COO-Alk
   - -N(R₁₁)-SO₂-Alk-NR₉R₁₀
   - -N(R₁₁)-SO₂-Alk
   - -N(R₁₁)-Alk-NR₅R₆
   - -N(R₁₁)-CO-Alk-NR₉R₁₀
   - -N(R₁₁)-CO-Alk
   - -N(R₁₁)-CO-CF₃
   - -NH-Alk-HetN
   - -O-Alk-NR₉R₁₀
   - -O-Alk-CO-NR₅R₆
   - -O-Alk-HetN
      in which n, m, Alk, R₅, R₆ and R₇ have the meaning given above for R₁, and
      - R₉ and R₁₀, which are identical or different, each represent a hydrogen atom or an alkyl radical of 1 to 5 carbon atoms,
      - R₁₁ represents a hydrogen atom or a radical -Alk-COOR₁₂ where R₁₂ represents a hydrogen atom, an alkyl radical of 1 to 5 carbon atoms or a benzyl radical,
      - HetN represents a 5- or 6-membered heterocycle containing at least one nitrogen atom and optionally another heteroatom chosen from nitrogen and oxygen,
   or R₃ and R₄ form together a 5- to 6-membered unsaturated heterocycle, provided, however, that when R₃ represents an alkoxy radical and R₄ represents a radical -O-Alk-NR₉R₁₀ or a hydroxyl radical, R₁ does not represent an alkoxy radical,
and their pharmaceutically acceptable salts and solvates.

The use of a compound of formula (I) is preferred in which:
- R₁ represents a hydroxyl radical, a linear or branched alkoxy radical of 1 to 5 carbon atoms, a carboxyl radical, an alkoxycarbonyl radical of 2 to 6 carbon atoms or a radical of formula:
   - -NR₅R₆
   - -NH-SO₂-Alk
   - -NH-SO₂-Ph
   - -NH-CO-Ph
   - -N(Alk)-CO-Ph
   - -NH-CO-NH-Ph
   - -NH-CO-Alk
   - -NH-CO₂-Alk
   - -O-(CH₂)ₙ-cAlk
   - -O-Alk-COOR₇
   - -O-Alk-O-R₈
   - -O-Alk-OH
   - -O-Alk-NR₅R₆
   - -O-Alk-CN
   - -O-(CH₂)ₙ-Ph
   - -O-Alk-CO-NR₅R₆
   - -CO-NH-(CH₂)ₘ-COOR₇
   - -CO-NH-Alk
   in which
   - Alk represents an alkyl radical or a linear or branched alkylene radical of 1 to 5 carbon atoms,
   - cAlk represents a cycloalkyl radical of 3 to 6 carbon atoms,
   - n represents an integer from 0 to 5,
   - m represents an integer from 1 to 5,
   - R₅ and R₆, which are identical or different, each represent a hydrogen atom, a linear or branched alkyl radical of 1 to 5 carbon atoms or a benzyl radical,
   - R₇ represents a hydrogen atom or an alkyl radical of 1 to 5 carbon atoms,
   - R₈ represents an alkyl radical of 1 to 5 carbon atoms or a radical -CO-Alk,
   - Ph represents a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
      - R₂ represents an alkyl radical of 1 to 5 carbon atoms, a trifluoromethyl radical, a cycloalkyl radical of 3 to 6 carbon atoms or a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
      - A represents a radical -CO- or -SO₂-,
      - R₃ and R₄, which are identical or different each represent a hydrogen atom, an alkoxy radical of 1 to 5 carbon atoms, an amino radical, a carboxyl radical, an alkoxycarbonyl radical of 2 to 6 carbon atoms, a nitro radical, a hydroxyamino radical, a radical of formula
   - -Alk-COOR₇
   - -NR₅R₆
   - -NH-Alk-COOR₇
   - -NH-COO-Alk
   - -N(R₁₁)-SO₂-Alk-NR₉R₁₀
   - -N(R₁₁)-SO₂-Alk
   - -N(R₁₁)-Alk-NR₅R₆
   - -N(R₁₁CO-Alk-NR₉R₁₀
   - -N(R₁₁)-CO-Alk
   - -N(R₁₁)-CO-CF₃
   - -NH-Alk-HetN
      in which n, m, Alk, R₅, R₆ and R₇ have the meaning given above for R₁, and
      - R₉ and R₁₀, which are identical or different, each represent a hydrogen atom or an alkyl radical of 1 to 5 carbon atoms,
      - R₁₁ represents a hydrogen atom or a radical -Alk-COOR₁₂ where R₁₂ represents a hydrogen atom, an alkyl radical of 1 to 5 carbon atoms or a benzyl radical,
      - HetN represents a 5- or 6-membered heterocycle containing at least one nitrogen atom and optionally another heteroatom chosen from nitrogen and oxygen,
   and its pharmaceutically acceptable salts and solvates.

The use of a compound of formula (I) is particularly preferred in which:
- R₁ represents an alkoxy radical of 1 to 5 carbon atoms, a carboxyl radical, a radical -O-Alk-COOH in which Alk represents a linear or branched alkylene radical of 1 to 5 carbon atoms, a radical of formula -O-Alk-Ph in which Alk represents an alkylene radical of 1 to 5 carbon atoms and Ph represents a phenyl radical which is optionally substituted with one or more halogen atoms or with one or more alkoxy radicals of 1 to 5 carbon atoms or with one or more carboxyl radicals, a radical of formula -NH-CO-Ph, a radical of formula -NH-SO₂-Ph or a radical of formula -NH-CO-NH-Ph,
- R₂ represents an alkyl radical of 1 to 5 carbon atoms,
- A represents a radical -CO-,
- R₃ and R₄, which are different, each represent a hydrogen atom, an alkoxy radical of 1 to 5 carbon atoms, an amino radical, a carboxyl radical or an alkoxycarbonyl radical of 2 to 6 carbon atoms,
and its pharmaceutically acceptable salts and solvates.

Among the compounds of formula (I), the 2-amino-5-[(1-methoxy-2-methylindolizin-3-yl)carbonyl]benzoic acid, its pharmaceutically acceptable salts and solvates can be used. The formula of this compound is as follows:

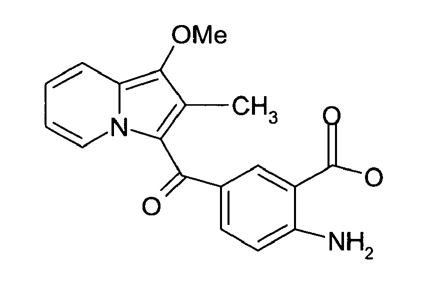

According to another of its features, the subject of the present invention is also a pharmaceutical composition containing, as active ingredient, a compound of formula (I) defined above or one of its pharmaceutically acceptable salts or solvates, in combination with one or more inert and appropriate excipients.
The said excipients are chosen according to the pharmaceutical dosage form and the desired mode of administration: oral, sublingual, subcutaneous, intra-articular, intramuscular, intravenous, transdermal, transmucosal, local or rectal.
In the pharmaceutical compositions of the present invention for oral administration, the active ingredients may be administered in a unit form for administration, as a mixture with conventional pharmaceutical carriers. The appropriate unit forms for administration comprise, for example, tablets, which are optionally scored, gelatine capsules, powders, granules and oral solutions or suspensions, drops and injectable solutions.

When a solid composition in the form of tablets is prepared, the main active ingredient is mixed with a pharmaceutical vehicle such as gelatine, starch, lactose, magnesium stearate, talc, gum Arabic and the like.
It is possible to coat the tablets with sucrose or other appropriate materials, or alternatively it is possible to treat them so that they have a prolonged or delayed activity and they continuously release a predetermined quantity of active ingredient.
A preparation in the form of gelatine capsules is obtained by mixing the active ingredient with a diluent and pouring the mixture obtained in soft or hard gelatine capsules.
A preparation in syrup or elixir form may contain the active ingredient together with a sweetener, preferably calorie-free, methylparaben and propylparaben as antiseptics, a taste enhancer and an appropriate colouring.
Water-dispersible powders or granules may contain the active ingredient as a mixture with dispersing agents, wetting agents or suspending agents, such as polyvinylpyrrolidone, and with sweeteners or flavour corrigents.
The active ingredient may also be formulated in the form of microcapsules, optionally with one or more carriers or additives.
In the pharmaceutical compositions according to the present invention, the active ingredient may also be in the form of an inclusion complex in cyclodextrins, their ethers or their esters.

The pharmaceutical composition according to the invention may be used in the treatment of degenerative joint diseases, such as osteoarthritis, spondyloses, cartilage loss following joint trauma or a relatively long period of joint immobilization following meniscus or patella injuries or ligament ruptures, and chronic diseases of the locomotory apparatus such as inflammatory, immunological or metabolism-associated acute and chronic arthritides, arthropathies, myalgias and disturbances of bone metabolism.

The quantity of active ingredient to be administered depends, as always, on the degree of progression of the disease and the age and weight of the patient.
The compositions according to the invention, for oral administration, therefore contain recommended doses of 0.01 to 700 mg

The following experimental data illustrate the present invention without limiting its scope.

### The effect of an FGF-antaqonist on the degree of knee joint damage in STR/1N mice, which develop osteoarthritis spontaneously

The experiment was performed according to the regulations of the German Animal Protection Law. Fifty 9-week old male STR/1N mice (strain bred and maintained at sanofi-aventis, Dept. of Laboratory Animal Safety and Welfare, D-65926 Frankfurt/Main, Germany) were randomized to 2 experimental groups (n=25/group). The animals were housed individually in standard mouse cages (room temperature 20 - 22°C; relative humidity 55-75%; 12 hours light/dark cycle) with free access to the powdered mouse standard food (Sniff R/M-H, Soest, Germany) and tap water. To achieve sustained drug exposure, the sodium 2-amino-5-[(1-methoxy-2-methylindolizin-3-yl)carbonyl]benzoic acid monohydrate was blended with the standard food at concentrations of 300 ppm (0.3 g/kg food) and administered to one experimental group of 25 mice ad libitum. The control group of 25 mice received unsupplemented standard food.
Following the 6-week treatment period the animals were sacrificed by CO₂ inhalation, their left knee joints were removed and decalcified for 3 days with formic acid (decalcifier, Shandon, Frankfurt/M., Germany), fixed in formalin and embedded in paraffin. Four sequential coronal sections of the complete knee joints (7 µm, 0.1 mm apart), stained with Hematoxyline-Eosine and Saffranin-O were evaluated by a blinded observer for the degree of histopathological joint alterations by using a semi-quantitative score as described in Table 1.

**Table 1: Semiquantitative Histopathological Score of Osteoarthritic Joint Damage in STR/1N Mice**

| MATRIX STRUCTURE | | CELLULARITY | |
|---|---|---|---|
| 0 | normal | 0 | normal |
| 1 | surface irregularities | 2 | reduced |
| 3 | superficial fibrillation | 5 | strongly reduced |
| 6 | clefts in deep zones | 8 | total loss of cells |
| 8 | complete loss of cartilage | | |

| MATRIX STAINING | | SUBCHONDRAL BONE STRUCTURE | |
|---|---|---|---|
| 0 | normal - slightly reduced | | |
| 1 | homogeneously reduced in deep layers | 0 | normal |
| | | 3 | remodelling processes |
| 3 | reduced in the interterritorial matrix, heterogeneous | 8 | thickening, strong sclerosis |
| 6 | severily reduced | | |
| 8 | no staining | | |
| Multiplied by Stages 1- 4 (according to % involvement of joint area) | | | |
| MAXIMAL TOTAL JOINT SCORE = 128 | | | |

The osteoarthritis joint damage was assessed by a semi-quantitative histopathological score. The STR/1N mice developed moderate osteoarthritis in the medial tibial plateau of the knee joint. The compound tested was well tolerated and significantly reduced the degree of histopathological joint damage damage by 32%.

| Group | Number of Animals | Histopathological Score Mean (SEM) |
|---|---|---|
| Non-treated Control | 25 | 34.3 (3.8) |
| sodium 2-amino-5-[(1-methoxy-2-methylindolizin-3-yl)carbonyl]benzoic acid monohydrate | 24 | 23.5 (3.7)* |

| | | |
|---|---|---|
| *p = 0.0118; Mann Whitney Test | | |

## Claims

1. Use of a 1,2,3-substituted indolizine derivatives of the following formula (I): 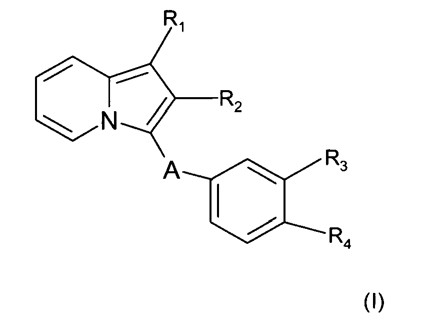 in which
- R₁ represents a hydroxyl radical, a linear or branched alkoxy radical of 1 to 5 carbon atoms, a carboxyl radical, an alkoxycarbonyl radical of 2 to 6 carbon atoms or a radical of formula:
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-C(NH₂):NOH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
in which
• Alk represents an alkyl radical or a linear or branched alkylene radical of 1 to 5 carbon atoms,
• cAlk represents a cycloalkyl radical of 3 to 6 carbon atoms,
• n represents an integer from 0 to 5,
• m represents an integer from 1 to 5,
• R₅ and R₆, which are identical or different, each represent a hydrogen atom, a linear or branched alkyl radical of 1 to 5 carbon atoms or a benzyl radical,
• R₇ represents a hydrogen atom or an alkyl radical of 1 to 5 carbon atoms,
• R₈ represents an alkyl radical of 1 to 5 carbon atoms or a radical -CO-Alk,
• Ph represents a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
- R₂ represents a hydrogen atom, an alkyl radical of 1 to 5 carbon atoms, an alkyl halide radical of 1 to 5 carbon atoms containing 3 to 5 halogen atoms, a cycloalkyl radical of 3 to 6 carbon atoms or a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
- A represents a radical -CO-, -SO- or -SO₂-,
- R₃ and R₄, which are identical or different, each represent a hydrogen atom, an alkoxy radical of 1 to 5 carbon atoms, an amino radical, a carboxyl radical, an alkoxycarbonyl radical of 2 to 6 carbon atoms, a hydroxyl radical, a nitro radical, a hydroxyamino radical, a radical of formula
• -Alk-COOR₇
• -NR₅R₆
• -NH-Alk-COOR₇
• -NH-COO-Alk
• -N(R₁₁)-SO₂Alk-NR₉R₁₀
• -N(R₁₁)-SO₂-Alk
• -N(R₁₁)-Alk-NR₅R₆
• -N(R₁₁)-CO-Alk-N₉R₁₀
• -N(R₁₁)-CO-Alk
• -N(R₁₁)-CO-CF₃
• -NH-Alk-HetN
• -O-Alk-NR₉R₁₀
• -O-Alk-CO-NR₅R₆
• -O-Alk-HetN
in which n, m, Alk, R₅, R₆ and R₇ have the meaning given above for R₁, and
• R₉ and R₁₀, which are identical or different, each represent a hydrogen atom or an alkyl radical of 1 to 5 carbon atoms,
• R₁₁ represents a hydrogen atom or a radical -Alk-COOR₁₂ where R₁₂ represents a hydrogen atom, an alkyl radical of 1 to 5 carbon atoms or a benzyl radical,
• HetN represents a 5- or 6-membered heterocycle containing at least one nitrogen atom and optionally another heteroatom chosen from nitrogen and oxygen,
or R₃ and R₄ form together a 5- to 6-membered unsaturated heterocycle, provided, however, that when R₃ represents an alkoxy radical and R₄ represents a radical -O-Alk-NR₉R₁₀ or a hydroxyl radical, R₁ does not represent an alkoxy radical,
and its pharmaceutically acceptable salts and solvates,
for the preparation of a medicament intended for the treatment of degenerative joint diseases.

2. The use, as claimed in claim 1, wherein said degenerative joint diseases are chosen among osteoarthritis, spondyloses, cartilage loss following joint trauma or a relatively long period of joint immobilization following meniscus or patella injuries or ligament ruptures, and chronic diseases of the locomotory apparatus such as inflammatory, immunological or metabolism-associated acute and chronic arthritides, arthropathies, myalgias and disturbances of bone metabolism.

3. The use, as claimed in claim 1 or 2, wherein said degenerative joint diseases is osteoarthritis.

4. The use, as claimed in claim 1, 2 or 3, of a compound of formula (I), in which:
- R₁ represents a hydroxyl radical, a linear or branched alkoxy radical of 1 to 5 carbon atoms, a carboxyl radical, an alkoxycarbonyl radical of 2 to 6 carbon atoms or a radical of formula:
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
in which
• Alk represents an alkyl radical or a linear or branched alkylene radical of 1 to 5 carbon atoms,
• cAlk represents a cycloalkyl radical of 3 to 6 carbon atoms,
• n represents an integer from 0 to 5,
• m represents an integer from 1 to 5,
• R₅ and R₆, which are identical or different, each represent a hydrogen atom, a linear or branched alkyl radical of 1 to 5 carbon atoms or a benzyl radical,
• R₇ represents a hydrogen atom or an alkyl radical of 1 to 5 carbon atoms,
• R₈ represents an alkyl radical of 1 to 5 carbon atoms or a radical -CO-Alk,
• Ph represents a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
- R₂ represents an alkyl radical of 1 to 5 carbon atoms, a trifluoromethyl radical, a cycloalkyl radical of 3 to 6 carbon atoms or a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
- A represents a radical -CO- or -SO₂-,
- R₃ and R₄, which are identical or different each represent a hydrogen atom, an alkoxy radical of 1 to 5 carbon atoms, an amino radical, a carboxyl radical, an alkoxycarbonyl radical of 2 to 6 carbon atoms, a nitro radical, a hydroxyamino radical, a radical of formula
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁-CO-Alk-NR₉R₁₀
• -N(R₁₁)-CO-Alk
• -N(R₁₁)-CO-CF₃
• -NH-Alk-HetN
in which n, m, Alk, R₅, R₆ and R₇ have the meaning given above for R₁, and
• R₉ and R₁₀, which are identical or different, each represent a hydrogen atom or an alkyl radical of 1 to 5 carbon atoms,
• R₁₁ represents a hydrogen atom or a radical -Alk-COOR₁₂ where R₁₂ represents a hydrogen atom, an alkyl radical of 1 to 5 carbon atoms or a benzyl radical,
• HetN represents a 5- or 6-membered heterocycle containing at least one nitrogen atom and optionally another heteroatom chosen from nitrogen and oxygen,
and its pharmaceutically acceptable salts and solvates.

5. The use, as claimed in claim 1, 2, 3 or 4, of a compound of formula (I), in which:
- R₁ represents an alkoxy radical of 1 to 5 carbon atoms, a carboxyl radical, a radical -O-Alk-COOH in which Alk represents a linear or branched alkylene radical of 1 to 5 carbon atoms, a radical of formula -O-Alk-Ph in which Alk represents an alkylene radical of 1 to 5 carbon atoms and Ph represents a phenyl radical which is optionally substituted with one or more halogen atoms or with one or more alkoxy radicals of 1 to 5 carbon atoms or with one or more carboxyl radicals, a radical of formula -NH-CO-Ph, a radical of formula -NH-SO₂-Ph or a radical of formula
- NH-CO-NH-Ph,
- R₂ represents an alkyl radical of 1 to 5 carbon atoms,
- A represents a radical -CO-,
- R₃ and R₄, which are different, each represent a hydrogen atom, an alkoxy radical of 1 to 5 carbon atoms, an amino radical, a carboxyl radical or an alkoxycarbonyl radical of 2 to 6 carbon atoms,
and its pharmaceutically acceptable salts and solvates.

6. The use, as claimed in anyone of claim 1 to 5, of a compound of formula (I), which is the 2-amino-5-[(1-methoxy-2-methylindolizin-3-yl)carbonyl]benzoic acid, and its pharmaceutically acceptable salts and solvates.

7. Pharmaceutical composition containing, as active ingredient, a compound of formula (I), a pharmaceutically acceptable salt thereof or a solvate thereof, according to any one of claims 1 to 6, in combination with one or more inert and appropriate excipients.

8. Pharmaceutical composition according to claim 7, which is useful in the treatment of degenerative joint diseases, such as osteoarthritis, spondyloses, cartilage loss following joint trauma or a relatively long period of joint immobilization following meniscus or patella injuries or ligament ruptures, and chronic diseases of the locomotory apparatus such as inflammatory, immunological or metabolism-associated acute and chronic arthritides, arthropathies, myalgias and disturbances of bone metabolism.
